(19) Europäisches Patentamt / European Patent Office / Office européen des brevets

(11) **EP 2 578 264 B1**

(12) **EUROPEAN PATENT SPECIFICATION**

(45) Date of publication and mention
of the grant of the patent:
**23.01.2019 Bulletin 2019/04**

(21) Application number: **11786693.9**

(22) Date of filing: **25.05.2011**

(51) Int Cl.:
**A61M 37/00** (2006.01)

(86) International application number:
**PCT/JP2011/062025**

(87) International publication number:
**WO 2011/148994 (01.12.2011 Gazette 2011/48)**

(54) **DEVICE HAVING ARRAY PROVIDED WITH FINE PROTRUSIONS**

VORRICHTUNG MIT EINEM ARRAY MIT FEINEN ERHEBUNGEN

DISPOSITIF POURVU D'UNE MATRICE DOTÉE DE FINES SAILLIES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **28.05.2010 JP 2010122997**

(43) Date of publication of application:
**10.04.2013 Bulletin 2013/15**

(73) Proprietor: **Hisamitsu Pharmaceutical Co., Inc.
Tosu-shi, Saga 841-0017 (JP)**

(72) Inventors:
• **TOKUMOTO Seiji**
**Tsukuba-shi**
**Ibaraki 305-0856 (JP)**
• **MATSUDO Toshiyuki**
**Tsukuba-shi**
**Ibaraki 305-0856 (JP)**
• **ISHITSUKA Shuji**
**Tsukuba-shi**
**Ibaraki 305-0856 (JP)**

(74) Representative: **Grünecker Patent- und
Rechtsanwälte
PartG mbB
Leopoldstraße 4
80802 München (DE)**

(56) References cited:
WO-A1-2006/062848    WO-A1-2010/001671
WO-A1-2010/074239    JP-A- 2006 149 818
JP-A- 2007 089 792    JP-A- 2007 130 417
JP-A- 2008 509 723    JP-A- 2008 522 731
US-A1- 2005 228 340    US-A1- 2008 208 134

## Description

### Technical Field

An embodiment of the present invention relates to an array provided with microprotrusions for administering an active ingredient via the skin.

### Background Art

Heretofore, one in which microprotrusions are disposed on a base (array provided with microprotrusions) has been known as a device for improving the transdermal absorption of drugs. The microprotrusions are aimed at puncturing the stratum corneum, which is the outermost layer of the skin, and various sizes or shapes have been proposed (see Patent Literature 1).

Moreover, various methods have also been proposed as to methods for applying drugs in the case of using the array provided with microprotrusions. In Patent Literature 2, it is described that: a drug is coated onto the surfaces of the microprotrusions; grooves or hollow portions for permeating a drug or a biogenic substance are disposed in the microprotrusions; a drug is mixed with the microprotrusions themselves; etc. Moreover, in Patent Literature 2, it is preferred that a reservoir medium should contain sugars, and it is also described that the reservoir medium particularly contains sugars for stabilization that forms glass (amorphous solid substance), such as lactose, raffinose, trehalose, or sucrose.

Furthermore, in Patent Literatures 3 and 4, there is the description that by setting the height of the microprotrusions to 10 μm to 3 mm and setting the shape of the tips of the microprotrusions to a flat shape or a rounded shape, the microprotrusions can administer cosmetics, pharmaceuticals, or compounds such as plastics attached to or contained in the protrusions while stretching the epidermis without penetrating the stratum corneum.

Moreover, Patent Literature 5 discloses a micro-needle patch comprising a micro-needle array having a support layer and micro-needles disposed on the support layer, whereby the micro-needles taper down to a flat tip.

### Citation List

### Patent Literature

Patent Literature 1: JP 2001-506904
Patent Literature 2: JP 2004-504120
Patent Literature 3: JP 2007-089792
Patent Literature 4: JP 2007-130417
Patent Literaure 5: US 2008/208134 A1

### Summary of Invention

### Technical Problem

In the case of administering an active ingredient to a sensitive portion in the skin, it is preferred not to completely penetrate the stratum corneum, in order to avoid the possibility that the skin gets damaged. The reason therefor is that when a through-hole is formed in the stratum corneum, the occurrence of skin irritation (erythema) or reduction in the water-holding capacity of the skin resulting from increase in water loss from the skin is caused. However, the patterns of shapes or heights of protrusions in the transdermal administration apparatus described in Patent Literatures 3 and 4 above are enormous, and it is not said to disclose the optimum device.

Thus, a device having an array provided with microprotrusions has been demanded, which is capable of administering an active ingredient to the skin without pain and with reliability while preventing damage to the stratum corneum of the skin.

### Solution to Problem

During the course of diligent study to solve the problems described above, the present inventors have found that merely setting the shape and density of microprotrusions may result in perforation of the corneum, and it is important to consider the extent to which the skin stretches.

Specifically, a device having an array provided with microprotrusions according to the present invention comprises: an array provided with microprotrusions, having a base and tapered microprotrusions each disposed on the base, each microprotrusion comprising a tip, a bottom and a surface, and tapering down toward the tip from the bottom connected to the base; and a holding member for placing the array provided with microprotrusions against the skin, wherein letting a distance from the tip to the bottom on an arbitrary side of each of the microprotrusions as a and letting the length of a second line segment prepared by projecting a first line segment representing the distance onto the base as b, a relationship of $1.0 < (a/b) \leq 7.5$ holds. The height of the microprotrusions is 50 to 300 $\mu$m, and the tip is flat and the area of the tip is 20 to 600 $\mu m^2$, wherein the microprotrusions are made of polyactic acid, and wherein an active ingredient is coated at least on a portion on the surfaces of the microprotrusions and/or on the base, said active ingredient being a low-molecular weight sodium hyaluronate with a molecular weight of 50000 to 110000.

According to such a device, the skin at a site coming into contact with each protrusion when the microprotrusion is placed thereagainst stretches by a value of a/b described above at the maximum. In this context, the value a/b represents the rate of stretching provided that the skin has been stretched completely along the side of the microprotrusion, and can be said to be the maximum rate of stretching of the skin by the microprotrusion. The present inventors have found that if this maximum rate of stretching can be kept at 7.5 or less, there is no risk of penetrating the stratum corneum of the skin. Specifically, by determining the shape of the microprotrusion so as to satisfy a relationship of $1.0 < (a/b) \leq 7.5$, an active ingredient can be administered to the skin without pain and with reliability via the stratum corneum that has thinned out by stretching, with damage to the stratum corneum prevented.

A device having an array provided with microprotrusions according to another embodiment not being part of the present invention comprises: an array provided with microprotrusions, having a base and microprotrusions; and a holding member for placing the array provided with microprotrusions against the skin, wherein the microprotrusions stretch the skin by 1.01 to 3.0 times. According to such a device, since the rate of stretching of the skin can be kept at 1.01 to 3.0, an active ingredient can be administered to the skin without pain and with reliability via the stratum corneum that has thinned out by stretching, with damage to the stratum corneum prevented.

In the device having an array provided with microprotrusions according to the invention, the microprotrusions are made of polylactic acid. Since the polylactic acid is biodegradable, in this case, burdens on the skin, etc., can be reduced even if the microprotrusions remain on the skin by breaking or the like. Moreover, the polylactic acid is also advantageous in terms of antigenicity and the unit price of a material.

In the device having an array provided with microprotrusions according to a further alternative embodiment not being part of the invention, the height of the microprotrusions may be 20 to 400 $\mu$m, and the width of the bottom may be 10 to 200 $\mu$m.

In the device having an array provided with microprotrusions according to a further alternative embodiment, the density of the microprotrusions may be 100 to 10000 protrusions/$cm^2$.

In the device having an array provided with microprotrusions according to the invention, the tip is flat, and the area of the tip is 20 to 600 $\mu m^2$. In this case, since pressure against the skin contacted with the tip of the microprotrusion is reduced, damage to this site can be avoided more reliably.

In the device having an array provided with microprotrusions according to a further alternative embodiment not being part of the invention, the tip may be rounded, and the radius of curvature of the tip may be 2 to 100 $\mu$m. In this case, since pressure against the skin contacted with the tip of the microprotrusion is reduced, damage to this site can be avoided more reliably.

In the device having an array provided with microprotrusions according to a further alternative embodiment not being part of the invention, the tip may be pointed, and the apical angle of the tip projected onto an arbitrary reference surface orthogonal to the base may be 16 degrees or larger. Since the value a/b can thereby be within the range of $1.0 < (a/b) \leq 7.5$, an active ingredient can be administered to the skin without pain and with reliability, with damage to the stratum corneum prevented even if the microprotrusions are pointed.

In the device having an array provided with microprotrusions according to the invention, an active ingredient is coated at least on a portion on the surfaces of the microprotrusions and/or on the base.

In the device having an array provided with microprotrusions according to a further alternative embodiment, the microprotrusions may not penetrate the stratum corneum of the skin.

In the device having an array provided with microprotrusions according to a further alternative embodiment, the holding member may have a support and a pressure-sensitive adhesive layer stacked on the support, and the array provided with microprotrusions may be placed on the pressure-sensitive adhesive layer. By disposing the array on the pressure-sensitive adhesive layer, the array can be placed easily and reliably against the skin.

**Advantageous Effects of Invention**

According to the present invention, letting a distance from the tip to the bottom on a side of each microprotrusion as a and letting the length of a second line segment prepared by projecting a first line segment representing the distance onto the base as b, a relationship of 1.0 < (a/b) ≤ 7.5 holds. By thus determining the shape of the microprotrusion, an active ingredient can be administered to the skin without pain and with reliability, with damage to the stratum corneum of the skin prevented.

**Brief Description of Drawings**

Figure 1 is a perspective view showing one example of an array provided with microprotrusions.
Figure 2 is a sectional view taken along the II-II line in Figure 1.
Figure 3(a) is a perspective view of a conical microprotrusion; Figure 3(b) is a sectional view taken along the B-B line in Figure 3(a); Figure 3(c) is a perspective view of a quadrangular pyramidal microprotrusion; and Figure 3(d) is a sectional view taken along the D-D line in Figure 3(c).
Figures 4(a) to 4(c) are each a diagram showing one example of a method for coating the microprotrusions.
Figure 5 is a graph showing water loss in Example 3.
Figure 6 is a graph showing change in impedance in Example 4.
Figure 7 is a diagram schematically showing the state in which the skin has been stretched incompletely along the microprotrusion.
Figure 8 is a plane view of a device having an array provided with microprotrusions according to an embodiment.
[Figure 9] Figure 9 is a sectional view taken along the IX-IX line in Figure 8.
Figure 10 is a graph showing change in brightness in Example 8.

**Description of Embodiments**

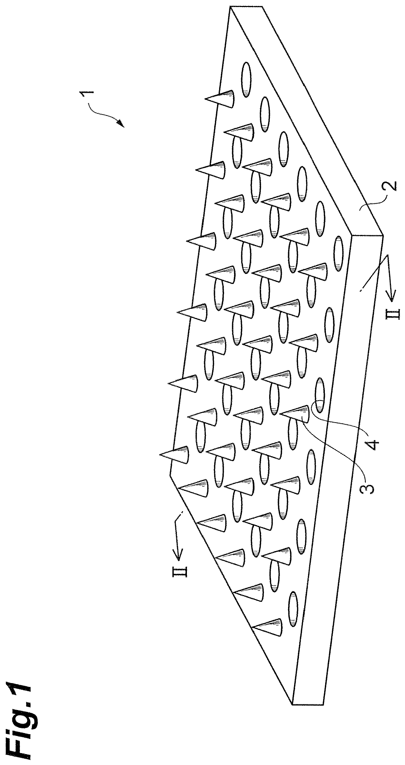

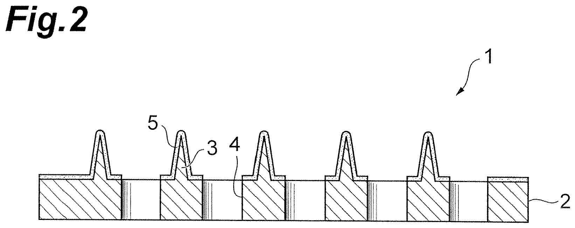

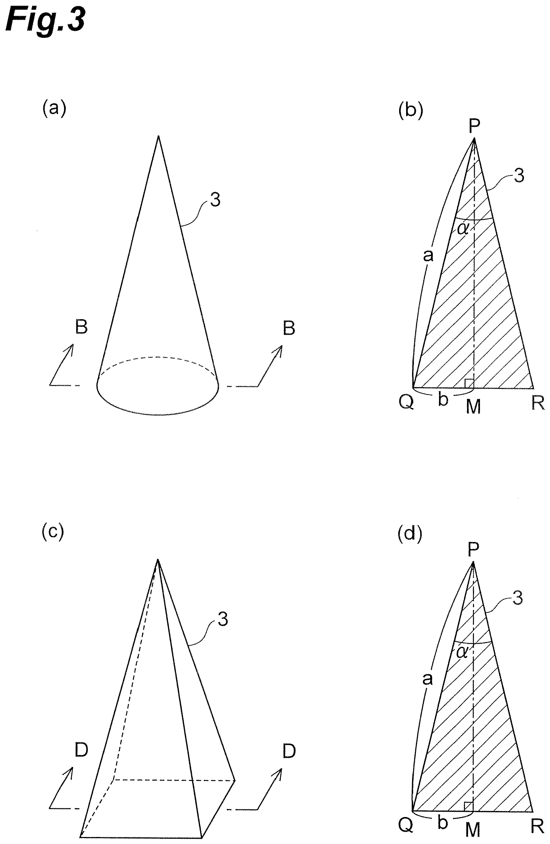

Hereinafter, embodiments of the present invention will be described in detail with reference to the accompanying drawings. Incidentally, in the description of the drawings, the same reference signs will be used to designate the same or similar components, so that the description will be omitted.
First, the constitution of an array 1 provided with microprotrusions (hereinafter, also simply referred to as an "array 1") will be described. In the present specification, the "array" is merely the name of an instrument. Figure 1 is a perspective view showing one example of the array 1 according to an embodiment. Figure 2 is a sectional view taken along the II-II line in Figure 1. Figure 3 is a perspective view and a sectional view of a microprotrusion 3.
As shown in Figure 1, the array 1 comprises a base 2 and a plurality of microprotrusions 3 arranged in a two-dimensional pattern on the base 2.
The base 2 is a foundation for supporting the microprotrusions 3. In the base 2, a plurality of through-holes 4 are formed so as to be arranged in a two-dimensional pattern. The microprotrusions 3 and the through-holes 4 are alternately arranged in the diagonal direction of the base 2. By means of the through-holes 4, it becomes possible to administer a biologically active ingredient from the back of the base 2. As a matter of course, a base free from such through-holes may be used. The area of the base 2 may be 0.5 cm$^2$ to 300 cm$^2$, may be 1 cm$^2$ to 100 cm$^2$, or may be 1 cm$^2$ to 50 cm$^2$. A base of the desired size may be constituted by connecting several individuals of this base 2.
Each microprotrusion 3 is a microstructure, and its height (length) h is, for example, 20 to 400 μm. In this context, the reason why the length of the microprotrusion 3 is set to 20 μm or larger is that the transdermal administration of an active ingredient is secured, and the reason for setting to 400 μm or smaller is that the microprotrusion is prevented more reliably from piercing the corneum of the skin. Alternatively, if the length of the microprotrusion 3 is 300 μm or smaller, an active ingredient in an amount that should enter intradermally can be administered efficiently. The length of the microprotrusion 3 may be 50 to 300 μm.
In this context, the microprotrusion is a tapered structure tapering down toward the tip from the bottom connected to the base 2 and means a needle shape in a broad sense or a structure containing a needle shape. As a matter of course, the microprotrusion is not limited to the needle shape having a sharp tip and also includes a shape free from a pointed end. In the case where the microprotrusion 3 has a conical structure, the diameter at its base may be on the order of 5 to 250 μm or may be 10 to 200 μm. Although conical microprotrusions 3 are shown in Figure 1, microprotrusions in a polygonal pyramidal shape such as a quadrangular pyramidal shape may be used.
Letting a distance from the tip to the bottom on a side (along a side) of the microprotrusion 3 as a and letting the length of a line segment prepared by projecting a line segment representing the distance a onto the base 2 as b, the relationship represented by the following formula (1) may hold:

$$1.0 < (a/b) \leq 7.5 \ ... \ (1)$$

As an example, the case where the microprotrusion 3 is conical/quadrangular pyramidal is shown in Figure 3. In the case where the microprotrusion 3 is conical as shown in Figure 3(a), a line segment representing a distance a from a tip P to the bottom on its side is an oblique side PQ in a triangle PQR (sectional view including the tip P) of Figure 3(b). Moreover, a line segment prepared by projecting the oblique side PQ onto the base 2 along a direction orthogonal to the base 2 is a line segment QM in the triangle PQR. In this context, a point M is the foot of a perpendicular from the point P to the base QR. Even if the microprotrusion 3 is quadrangular pyramidal as shown in Figure 3(c), the same as in the conical case can hold true therefor as shown in Figure 3(d).

In this context, the value a/b is the rate of stretching provided that the skin in a normal state has been stretched completely along the side of the microprotrusion 3, i.e., an index representing by what times the skin is stretched by the microprotrusion 3 at the maximum, and can be said to be the maximum rate of stretching of the skin by the microprotrusion 3. In the examples of Figure 3, the maximum rate of stretching of the skin may satisfy the above formula (1) in the case where the skin at a site along the line segment QM has been stretched along the oblique side PQ by the application of the array 1. This is because if the rate of stretching of the skin is kept at this level, damage to the stratum corneum can be avoided more reliably when the array 1 is placed thereagainst. The rate of stretching may be 1.01 to 3.0 or may be 1.01 to 2.0.

In the case where the microprotrusion 3 is conical or pyramidal, the apical angle $\alpha$ (see Figures 3(c) and 3(d)) of the tip projected onto an arbitrary reference surface orthogonal to the base 2 may be 16 degrees or larger (less than 180 degrees). Specifically, the minimum value of the apical angle $\alpha$ of the tip projected onto the arbitrary reference surface may be 16 degrees or larger. Since the maximum rate of stretching of the skin can be kept within the range of the above formula (1) by thus adjusting the apical angle, damage attributed to the microprotrusion can be avoided more reliably.

The above formula (1) holds not only for the examples of Figure 3, but also holds even if the microprotrusion is arbitrarily pyramidal or is arbitrarily oblique conical. However, it is required that the formula (1) should hold for an arbitrary side if the microprotrusion is pyramidal and that the formula (1) should hold for an arbitrary generatrix if conical.

The microprotrusion may not be conical and pyramidal, and, for example, the tip may be flat or may be rounded. In the case where the tip is flat, the area of the flat portion may be 20 to 600 $\mu$m or may be 50 to 250 $\mu m^2$. Alternatively, in the case where the tip is rounded, the radius of curvature of the tip may be 2 to 100 $\mu$m or may be 5 to 30 $\mu$m. Even if the microprotrusion has such a shape, the above formula (1) holds. Since pressure against the skin contacted with the tip of the microprotrusion is reduced by thus processing the tip of the microprotrusion, damage to this site can be avoided more reliably.

Although the microprotrusions 3 may be ones that do not penetrate the stratum corneum of the skin in usual use, it is also possible that some microprotrusions 3 penetrate the stratum corneum as long as there is no inconvenience such as inflammation from the viewpoint of skin beauty. Specifically, the epidermis thins out by stretching by the microprotrusions 3, and an active ingredient permeates the epidermis that has been rendered permeable, though it is possible that a portion of the active ingredient enters into the skin from the pierced corneum.

Regarding the density of the microprotrusions 3, typically, spacing is given so that a density of approximately 1 to 10 per mm is provided as to the row of needles. In general, adjacent rows are spaced from each other by a distance substantially equal to the space between the needles in each row, and have a density of 100 to 10000 needles per $cm^2$. The density of the microprotrusions 3 may be 200 to 5000 protrusions or may be 300 to 2000 protrusions or 400 to 850 protrusions.

Examples of the material of the base 2 or the microprotrusions 3 include silicon, silicon dioxide, ceramics, metals (stainless, titanium, nickel, molybdenum, chromium, cobalt, etc.), and synthetic or natural resin materials, but include biodegradable polymers such as polylactic acid, polyglycolide, polylactic acid-co-polyglycolide, pullulan, caprolactone, polyurethane, and polyanhydrides, and synthetic or natural resin materials such as polycarbonate, polymethacrylic acid, ethylene vinyl acetate, polytetrafluoroethylene, and polyoxymethylene, which are non-degradable polymers, in consideration of the antigenicity of the microprotrusions and the unit price of the material. Moreover, hyaluronic acid, sodium hyaluronate, pullulan, dextran, dextrin or chondroitin sulfate, a cellulose derivative, and the like, which are polysaccharides may be used. Moreover, in an alternative embodiment not being part of the invention, one in which an active ingredient is mixed with the biodegradable resin may be used as the material of the base 2 and/or the microprotrusions 3.

The material of the microprotrusions 3 may be a biodegradable resin such as polylactic acid in consideration of breaking on the skin. Although a polylactic acid homopolymer of poly-L-lactic acid or poly-D-lactic acid, a poly-L/D-lactic acid copolymer, and a mixture thereof, etc. are included in the polylactic acid, any of these may be used. Moreover, the larger the average molecular weight of the polylactic acid becomes, the larger its strength becomes, and those of 40,000 to 100,000 can be used.

Examples of the method for producing the base 2 or the microprotrusions 3 include wet etching or dry etching using a silicon base, precision machining using metal or resin (electro-discharge machining, laser processing, grinding, hot embossing, injection molding, etc.), and machinery cutting. By these processing methods, the protrusions and the supporting portion are integrally molded. Examples of the method for rendering the protrusions hollow include a method of performing secondary processing by laser processing or the like after preparation of the protrusions.

In a certain example, coating 5 with an active ingredient is provided on the base 2 and/or the microprotrusions 3. In the present example, the coating 5 is one in which a coating solution containing a polymer carrier having compatibility with the active ingredient is anchored to a portion or the whole of the microprotrusions 3 and/or surface of the base 2. Examples of the polymer carrier include carboxyvinyl polymers and polyethylene oxide described later, polyvinylpyrrolidone, polyvinyl alcohol, and cellulose derivatives. "Anchored" refers to maintaining the state in which the coating solution is almost evenly attached to an object. Immediately after coating, the coating solution is anchored in a dry state by a known drying method of air drying, vacuum drying, freeze drying, or a combination thereof, but is not limited to anchoring in a dry state because of also retaining a water content in equilibrium with a surrounding atmosphere, an organic solvent, or the like after transdermal administration.

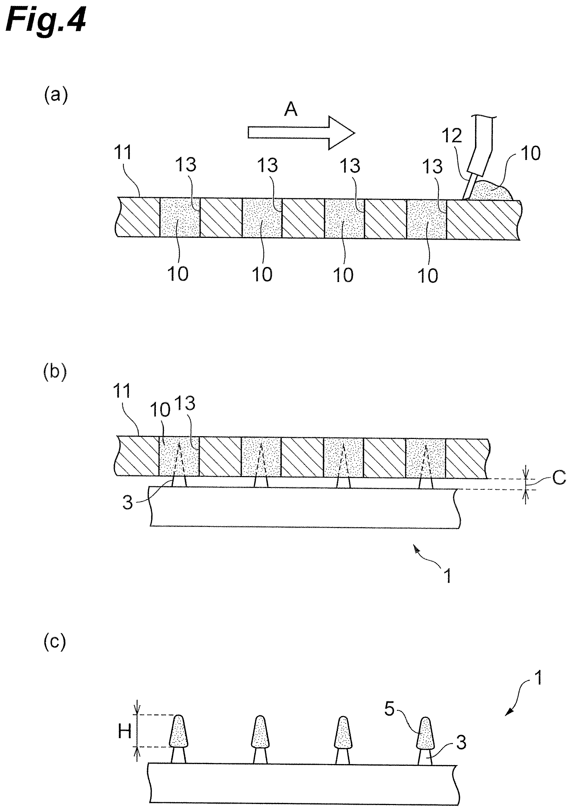

Figures 4(a) to 4(c) are each a diagram showing one example of the method for coating the microprotrusions 3. In this method, first, a coating solution 10, as shown in Figure 4(a), is swept in the direction of the arrow A with a spatula 12 on a mask sheet 11, so that the coating solution 10 is filled into openings 13. Subsequently, as shown in Figure 4(b), the microprotrusions 3 are inserted to the openings 13 of the mask sheet 11. Then, as shown in Figure 4(c), the microprotrusions 3 are pulled out of the openings 13 of the mask sheet 11. The coating 5 with the coating solution 10 is thereby provided on the microprotrusions 3. The coating 5 is anchored to the microprotrusions 3 by drying.

The range H of coating on each microprotrusion 3 is adjusted by the thickness of a clearance (gap) C or the mask sheet 11 shown in Figure 4(b). This clearance C is defined by a distance (base thickness is not involved) from the base of the microprotrusion 3 to the undersurface of the mask sheet 11 and set according to the tension of the mask sheet 11 and the length of the microprotrusion 3. The range of distance of the clearance C is, for example, 0 to 500 $\mu$m. In the case where the distance of the clearance C is 0, the whole of the microprotrusion 3 is coated. The range H of coating can be set to 0 to 500 $\mu$m and is usually 10 to 500 $\mu$m or may be on the order of 30 to 300 $\mu$m, though varying depending on the height h of the microprotrusion 3.

The thickness of the coating 5 on the base 2 and/or the microprotrusions 3 may be less than 50 $\mu$m, may be less than 25 $\mu$m, or may be 1 to 10 $\mu$m. In general, the thickness of the coating is an average thickness measured throughout the surface of the microprotrusion 3 after drying. The thickness of the coating can generally be increased by applying a plurality of films of the coating carrier, i.e., increased by repeating the coating step after coating carrier anchoring.

In performing coating on the base 2 and/or the microprotrusions 3, the temperature and humidity of the installation environment of an apparatus may be controlled at a constant level in order to minimize change in drug concentration and change in physical properties caused by the solvent volatilization of the coating agent. Either of decreasing of temperature or increasing of humidity, or both, may be controlled in order to prevent the evaporation of the solvent. The humidity at room temperature in the case of not controlling temperature may be 50 to 100% RH, may be 70 to 100% RH, or may be 90 to 100% RH, as a relative humidity. At 50% RH or less, the significant evaporation of the solvent occurs, and change in the physical properties of the coating solution occurs. A humidification system includes a vaporization system, a steam system, a water spray system, etc., but the humidification system is not particularly limited as long as the humid state of interest can be secured. With regard to a thickener to be mixed with the coating solution, a highly wettable or water-retaining water-soluble polymer that minimizes the volatility of the solvent may be selected.

The coating agent contains an active ingredient and purified water and/or a coating carrier. The coating carrier includes polyethylene oxide, hydroxymethyl cellulose, hydroxypropyl cellulose, hydroxypropyl methyl cellulose, methylcellulose, carboxymethyl cellulose, carmellose sodium, dextran, polyethylene glycol, polyvinyl alcohol, polyvinylpyrrolidone, pullulan, chondroitin sulfate, hyaluronic acid, sodium hyaluronate, dextrin, gum arabic, ethanol, isopropanol, methanol, propanol, butanol, propylene glycol, dimethyl sulfoxide, glycerin, N,N-dimethylformamide, polyethylene glycol, benzyl benzoate, sesame oil, soybean oil, lactic acid, benzyl alcohol, polysorbate 80, alpha thioglycerin, ethylenediamine, N,N-dimethylacetamide, thioglycolic acid, phenoxyethanol, etc.

A water-soluble polymer carrier having compatibility (property of uniformly mixing) with the active ingredient may be used as the coating carrier. Specifically, examples thereof include polyvinylpyrrolidone, polyvinyl alcohol, carboxyvinyl polymers, polyacrylic acid, sodium polyacrylate, polyoxyethylene polyoxypropylene glycol, Pluronic, polyethylene oxide, polyethylene glycol, propylene glycol, glycerin, butylene glycol, polyvinylacetamide, hydroxypropylcellulose, and pullulan. Particularly, examples thereof include carboxyvinyl polymers, polyethylene oxide, polyvinylpyrrolidone, hydroxypropylcellulose, pullulan, propylene glycol, glycerin, and butylene glycol.

The content of the coating carrier in the coating agent may be 0.1 to 70% by weight, may be 0.1 to 60% by weight, or may be 1 to 40% by weight. This coating carrier may need to be viscous to some extent so as not to drip, and requires approximately 100 to 100000 cps as a viscosity. This viscosity may be 500 to 60000 cps. The viscosity falls within this range, whereby it becomes possible to apply at one time the desired amount of the coating solution without depending on the material of the microprotrusions 3. Moreover, in general, the higher the viscosity becomes, the larger the amount of the coating solution tends to be.

A liquid composition used for coating the base 2 and/or the microprotrusions 3 is prepared by mixing a biocompatible carrier, a beneficial active ingredient to be delivered, and, in some cases, any coating auxiliary with a volatile liquid. The volatile liquid can be water, dimethyl sulfoxide, dimethylformamide, ethanol, isopropyl alcohol, and a mixture thereof. For example, water may be selected. The liquid coating solution or suspension can typically have 0.1 to 65% by weight of a beneficial biologically active ingredient concentration, and the concentration may be 1 to 40% by weight or 10 to 30% by weight. The coating may become an anchored state. A surfactant may be zwitterionic, amphoteric, cationic, anionic, or nonionic. For example, Tween 20 and Tween 80, other sorbitan derivatives, for example, sorbitan laurate, and alkoxylated alcohols, for example, laureth-4, may be used. For example, the addition of a surfactant is also effective for dissolving a larger amount of the active ingredient in the coating carrier.

Other known pharmaceutical auxiliaries may be added to the coating as long as they do not have harmful influence on the features of solubility and viscosity necessary for the coating and the properties and physical properties of dried coating.

The active ingredient used in the present disclosure is selected without particular limitations from the group consisting of antioxidants, free radical scavengers, humectants, depigmentation agents, fat controllers, UV-reflecting agents, wetting agents, antimicrobial agents, antiallergic drugs, anti-acne drugs, antiaging drugs, wrinkle defense drugs, bactericides, analgesics, antitussives, antipruritic drugs, local anesthetics, antialopecia agents, hair growth-promoting agents, hair growth-inhibiting agents, anti-dandruff agents, antihistaminic agents, keratolytic agents, anti-inflammatory drugs, refreshing drinks, therapeutic drugs, anti-infective drugs, preventive drugs for inflammation, antiemetics, anticholinergic drugs, vasoconstrictors, vasodilators, trauma healing aids, peptides, polypeptides, proteins, deodorants, antiperspirants, skin softeners, skin moisturizer solutions, softening agents, hair conditioners, hair softeners, hair moisturizers, tanning agents, skin-whitening agents, antifungal agents, depilatories, analgesic drugs for external use, drugs for counterirritation, drugs for hemorrhoids, insecticides, therapeutic drugs for poison ivy rash, therapeutic drugs for poison sumac rash, therapeutic drugs for burns, anti-diaper rash drugs, drugs for heat rash, skin lotions, vitamins, amino acids, amino acid derivatives, herb extracts, retinoid, flavonoid, sense markers, skin conditioners, hair lighteners, chelating agents, cellular turnover enhancers, coloring agents, sunscreens, anesthetics, immunostimulators, revitalizers, water absorbers, sebum absorbers, and mixtures thereof.

The active ingredient used in the present disclosure can also contain, for example, a plant preparation such as extracts or tinctures, for the treatment of local skin disease. Examples of the extracts or tinctures include oak bark extracts, walnut extracts, arnica tinctures, witch hazel extracts, *Plantago lanceolata* extracts, pansy extracts, thyme or sage extracts; St. John's wort tinctures, golden glow extracts, chamomile flower extracts, or calendula tinctures; and, for example, birch leaf extracts, nettle extracts, coltsfoot extracts, comfrey tinctures, horsetail extracts, aloe extracts, *Aesculus turbinata* and *Ruscus aculeatus* extracts, and arnica, calendula, and chili pepper extracts, for a care for heavily tired skin or damaged skin.

The amino acids as the active ingredient that may be used in the present disclosure include not only salts, esters, or acyl derivatives thereof but also amino acids obtained from the hydrolysis of various proteins. Examples of such amino acid drugs include: amphoteric amino acids such as alkylamidoalkylamines, stearyl acetyl glutamate, capryloyl silk amino acids, and capryloyl collagen amino acids; capryloyl keratin amino acids; capryloyl pea amino acids; cocodimonium hydroxypropyl silk amino acids; corn gluten amino acids; cysteine; glutamic acid; glycine; hair keratin amino acids; hair amino acids such as aspartic acid, threonine, serine, glutamic acid, proline, glycine, alanine, half-cystine, valine, methionine, isoleucine, leucine, tyrosine, phenylalanine, cysteic acid, lysine, histidine, arginine, cysteine, tryptophan, and citrulline; lysine; silk amino acids; wheat amino acids; and mixtures thereof.

The peptides, the polypeptides, and the proteins as the active ingredient that may be used in the present disclosure include, for example, polymers having a long chain whose number of carbon atoms is at least approximately 10, and a high molecular weight of, for example, at least 1000, and they are formed by the self-condensation of amino acids. Examples of such proteins include: collagen; deoxyribonuclease; iodized corn protein; keratin; milk protein; protease; serum protein; silk; sweet almond protein; wheat germ protein; wheat protein; alpha and beta helix of wheat protein or keratin protein; and hair proteins such as intermediate filament protein, high-sulfur content protein, ultrahigh-sulfur content protein, intermediate filament-associated protein, high-tyrosine protein, high-glycine/tyrosine protein, trichohyalin, and mixtures thereof.

Examples of the antiwrinkle ingredients that may be used in the present disclosure include hyaluronic acid, sodium hyaluronate, retinol (vitamin A), silybin peptides (HTC collagen, palmitoyl penta, peptide 3, and Argireline), amino acids, hydroxyproline, tocopheryl retinoate, ursolic acid, vitamin C derivatives, coenzyme Q10, astaxanthin, fullerene, polyphenols, alpha lipoic acid, soybean extracts, pullulan, active isoflavone, sugars, polysaccharides, glycerin, and glycerin derivatives. However, the antiwrinkle ingredients are not limited to these, and mixing is also possible.

Sodium hyaluronate is promising as a coating carrier and an antiwrinkle ingredient. Particularly, low-molecular-weight sodium hyaluronate whose molecular weight is on the order of 50000 to 110000 is preferable because of having higher adherence to the array provided with microprotrusions than that of high-molecular-weight sodium hyaluronate.

Examples of the vitamins used in the present disclosure include: vitamin B complex; vitamins A (e.g., vitamin A palmitate), C, D, E, and K and theirs derivatives, including thiamine, nicotinic acid, biotin, pantothenic acid, choline, riboflavin, vitamin B6, vitamin B12, pyridoxine, inositol, and carnitine; and provitamins such as panthenol (provitamin B5) and panthenol triacetate; and mixtures thereof.

Examples of the antimicrobial agents that may be used in the present disclosure include bacitracin, erythromycin, neomycin, tetracycline, chlortetracycline, benzethonium chloride, phenol, and mixtures thereof.

Examples of the skin softeners and the skin moisturizers that may be used in the present disclosure include mineral oils, lanolin, vegetable oils, isostearyl isostearate, glyceryl laurate, methyl gluceth-10, methyl gluceth-20, chitosan, and mixtures thereof.

Examples of the hair conditioners that may be used in the present disclosure include not only lipophilic compounds such as cetyl alcohol, stearyl alcohol, hydrogenated polydecene, and mixtures thereof, but also quaternary compounds such as behenamidopropyl PG-dimonium chloride, tricetyl ammonium chloride, dihydrogenated tallowamidoethyl hydroxyethylmonium methosulfate, and mixtures thereof.

Examples of the sunscreens that may be used in the present disclosure include butyl methoxydibenzoylmethane, octyl methoxycinnamate, oxybenzone, octocrylene, octyl salicylate, phenylbenzimidazole sulfonic acid, ethyl hydroxypropyl aminobenzoate, menthyl anthranilate, aminobenzoic acid, cinoxate, methoxycinnamic acid diethanolamine, glycerin aminobenzoate, titanium dioxide, zinc oxide, oxybenzone, Padimate O, red petrolatum, and mixtures thereof. The tanning agent that may be used in the present disclosure is dihydroxyacetone.

Examples of the skin-whitening agents that may be used in the present disclosure include hydroquinone and its derivatives, catechol and its derivatives, ascorbic acid and its derivatives, ellagic acid and its derivatives, kojic acid and its derivatives, tranexamic acid and its derivatives, resorcinol derivatives, placenta extracts, arbutin, oil-soluble licorice extracts, and mixtures thereof.

Examples of the anti-inflammatory analgesics that may be used in the present disclosure include acetaminophen, methyl salicylate, monoglycol salicylate, aspirin, mefenamic acid, flufenamic acid, indomethacin, diclofenac, alclofenac, diclofenac sodium, ibuprofen, ketoprofen, naproxen, pranoprofen, fenoprofen, sulindac, fenclofenac, clidanac, flurbiprofen, fentiazac, bufexamac, piroxicam, phenylbutazone, oxyphenbutazone, clofezone, pentazocine, mepirizole, and tiaramide hydrochloride. Examples of the steroidal anti-inflammatory analgesics that may be used together with the patch of the present disclosure include hydrocortisone, prednisolone, dexamethasone, triamcinolone acetonide, fluocinolone acetonide, hydrocortisone acetate, prednisolone acetate, methylprednisolone, dexamethasone acetate, betamethasone, betamethasone valerate, flumethasone, fluorometholone, and beclomethasone dipropionate.

Examples of the antihistaminic drugs that may be used in the present disclosure include diphenhydramine hydrochloride, diphenhydramine salicylate, diphenhydramine, chlorpheniramine hydrochloride, chlorpheniramine maleate, isothipendyl hydrochloride, tripelennamine hydrochloride, promethazine hydrochloride, and methdilazine hydrochloride. Examples of the local anesthetics that may be used together with the patch of the present disclosure include dibucaine hydrochloride, dibucaine, lidocaine hydrochloride, lidocaine, benzocaine, p-buthylaminobenzoic acid 2-(diethylamino) ethyl ester hydrochloride, procaine hydrochloride, tetracaine, tetracaine hydrochloride, chloroprocaine hydrochloride, oxyprocaine hydrochloride, mepivacaine, cocaine hydrochloride, piperocaine hydrochloride, dyclonine, and dyclonine hydrochloride.

Examples of the bactericides and the disinfectants that may be used in the present disclosure include thimerosal, phenol, thymol, benzalkonium chloride, benzethonium chloride, chlorhexidine, povidone iodine, cetylpyridinium chloride, eugenol, and trimethylammonium bromide. Examples of the vasoconstrictors that may be used together with the patch of the present disclosure include naphazoline nitrate, tetrahydrozoline hydrochloride, oxymetazoline hydrochloride, phenylephrine hydrochloride, and tramazoline hydrochloride. Examples of the hemostats that may be used together with the patch of the present disclosure include thrombin, phytonadione, protamine sulfate, aminocaproic acid, tranexamic acid, carbazochrome, carbazochrome sodium sulfonate, rutin, and hesperidin.

Examples of the chemotherapeutic drugs that may be used in the present disclosure include sulfamine, sulfathiazole, sulfadiazine, homosulfamine, sulfisoxazole, sulfisomidine, sulfamethizole, and nitrofurazone. Examples of the antibiotics that may be used together with the patch of the present disclosure include penicillin, methicillin, oxacilline, cephalothin, cephalodine, erythromycin, lincomycin, tetracycline, chlorotetracycline, oxytetracycline, methacycline, chloramphenicol, kanamycin, streptomycin, gentamycin, bacitracin, and cycloserine.

Examples of the antiviral drugs that may be used in the present disclosure include protease inhibitors, thymidine kinase inhibitors, sugar or glycoprotein synthesis inhibitors, constituent protein synthesis inhibitors, adherence and adsorption inhibitors, and nucleoside analogs such as acyclovir, penciclovir, valaciclovir, and ganciclovir.

Examples of the hair growth stimulants or hair restorers that may be used in the present disclosure include minoxidil, carpronium chloride, pentadecanoic acid glyceride, tocopherol acetate, piroctone olamine, glycyrrhizic acid, isopropylmethylphenol, hinokitiol, *Swertia japonica* extracts, ceramide and precursors, nicotinic acid amide, and chili pepper tinctures.

Examples of the beauty active ingredients that may be used in the present disclosure include D-alpha tocopherol, DL-alpha tocopherol, D-alpha-tocopheryl acetate, DL-alpha-tocopheryl acetate, ascorbyl palmitate, vitamin F and vitamin F glyceride, vitamin D, vitamin D2, vitamin D3, retinol, retinol ester, retinyl palmitate, retinyl propionate, beta carotene, coenzyme Q10, D-panthenol, farnesol, farnesyl acetate; jojoba oil and black currant oil abundantly contained in essential fatty acid; 5-n-octanoyl salicylic acid and its esters, salicylic acid and its esters; alkyl esters of alpha hydroxy acids such as citric acid, lactic acid, and glycolic acid; asiatic acid, madecassic acid, asiaticoside, total extracts of *Centella asiatica,* beta glycyrrhetinic acid, alpha bisabolol, ceramide such as 2-oleoylamino-1,3-octadecane; phytantriol, phytoplankton-derived phospholipid abundantly contained in poly-unsaturated essential fatty acid, ethoxyquin; rosemary extracts, balm extracts, quercetin, dry microalgal extracts, anti-inflammatory drugs such as steroidal anti-inflammatory drugs, and biochemical stimulants such as hormones or fats and/or compounds attributed to the synthesis of proteins.

The vitamin C used in the present disclosure promotes collagen (connective tissue) synthesis, lipid (fat) and carbohydrate metabolism, and neurotransmitter synthesis. The vitamin C is also essential for the optimum maintenance of the immune system. The vitamin C is harmful to a wide range of cancer cells, particularly, melanoma. Tyrosine oxidase, which catalyzes the aerobic activity of tyrosine that is converted to melanin and other pigments, is also prevented in the presence of vitamin C from being activated. The vitamin C has been found to be effective for catalyzing immune response to infections with many viruses and bacteria. In addition to many applications described above, the vitamin C is essential for collagen synthesis and trauma treatment. The patch to which the present disclosure is applied may contain a combination of vitamin C, vitamin E, and other ingredients such as humectants, collagen synthesis promoters, and facial scrubs.

The skin conditioner ingredients in the present disclosure include mineral oils, Vaseline, vegetable oils (e.g., soybean oil or maleated soybean oil), dimethicone, dimethicone copolyol, cationic monomers and polymers (e.g., guar hydroxypropyltrimonium chloride and distearyl dimethyl ammonium chloride), and mixtures thereof. Examples of the humectants include polyols such as sorbitol, glycerin, propylene glycol, ethylene glycol, polyethylene glycol, polypropylene glycol, 1,3- butanediol, hexylene glycol, isoprene glycol, xylitol, fructose, and mixtures thereof.

These active ingredients may be used alone or may be used in combination of two or more types, and as a matter of course, active ingredients in any form of inorganic salts or organic salts are also included as long as they are pharmaceutically acceptable salts. Moreover, although the active ingredient is basically incorporated in the coating carrier, the active ingredient can also be supplied later via the through-holes 4 formed in the base 2 without being incorporated in the coating carrier. Alternatively, the active ingredient is directly applied to the skin, and then, the array 1 provided with microprotrusions can also be placed against the same site on the skin. In this case, it becomes possible to promote the permeation of the active ingredient into the skin by virtue of the effect of stretching the skin and the effect of ODT (occlusive dressing therapy) on the skin.

An auxiliary instrument for fixing the array 1 may be used in administration using the array 1 provided with microprotrusions. However, direct administration by hand pressing is more preferable than a device that generates high collision energy as described in JP 2004-510535. When the array 1 comes in contact with the skin, administration is performed with a force of 1.0 to 10 kg or administration is performed with a force of 1.0 to 7 kg or with a force of 1.0 to 4 kg. Moreover, the administration time with this force is not so long and is on the order of a few seconds to 120 minutes at the longest. The administration time may be within 60 minutes or may be within 15 minutes. However, it is also possible to subsequently continue to administer the active ingredient by means of the holding member.

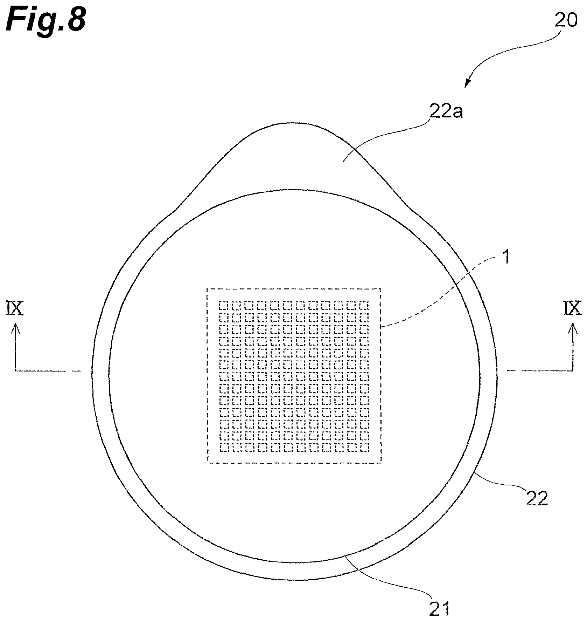

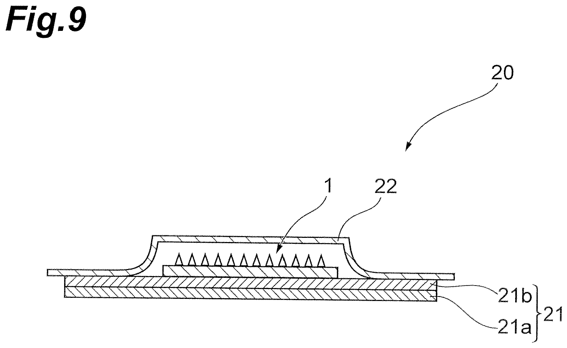

Next, the constitution of a device comprising an array 1 provided with microprotrusions and a holding member (device having an array provided with microprotrusions) will be described using Figures 8 and 9. Figure 8 is a plane view showing a device 20 having an array provided with microprotrusions (hereinafter, also simply referred to as a "device 20") according to the disclosure. Figure 9 is a sectional view taken along the IX-IX line in Figure 8.

The device 20 comprises the array 1, a holding member 21, and a release liner 22.

The holding member 21 is a substantially round tape for placing the array 1 against the skin over a predetermined time and comprises a support 21a and a pressure-sensitive adhesive layer 21b stacked on one surface of the support 21a. In this context, the meaning of puncturing is also included in the "placing against" in the present specification.

The support 21a needs only to be a support as usually used in medical plasters, tapes for fixation, and patches. Specifically, in addition to a synthetic resin such as polyethylene, polypropylene, polybutadiene, ethylene-vinyl acetate copolymers, acetic acid-vinyl chloride copolymers, polyvinyl chloride, polyamide, polyester, nylon, cellulose derivatives, or polyurethane, woven fabric (including knitted fabric), nonwoven fabric, or the like can also be used as the support 21a.

Although there is no particular limitation on the pressure-sensitive adhesive constituting the pressure-sensitive adhesive layer 21b as long as it is a pressure-sensitive adhesive as usually used in medical plasters, tapes for fixation, and patches, examples thereof include rubber-based, acrylic, silicon-based, and water-soluble adhesive bases.

One type selected from among natural rubbers, styrene-butadiene rubbers, styrene-isoprene-styrene block copolymers, styrene-butadiene-styrene block copolymers, polyisoprene, polybutene, polyisobutylene, and butyl rubbers, or the combined use of two or more types of these is possible as a rubber component in the rubber-based adhesive base.

Although any of those pharmaceutically acceptable may be used as the acrylic adhesive base, examples thereof include pressure-sensitive adhesives such as acrylic acid-acrylic acid octyl ester copolymers, 2-ethylhexyl acrylate-vinylpyrrolidone copolymer solutions, acrylic acid ester-vinyl acetate copolymers, 2-ethylhexyl acrylate-2-ethylhexyl methacrylate-dodecyl methacrylate copolymers, methyl acrylate-2-ethylhexyl acrylate copolymer resin emulsions, acrylic polymers contained in acryl resin alkanol amine solutions.

The water-soluble adhesive bases include gelatin, pectin, agarose, alginate, xanthan gum, dextrin, methylcellulose, ethylcellulose, hydroxyethylcellulose, carboxymethylcellulose, polyvinyl alcohol, polyvinylpyrrolidone, polyethylene glycol, maleic anhydride copolymers, polyacrylic acid and its salts, etc., and further examples thereof include natural polymers such as cross-linked forms thereof, or their modified forms, and synthetic polymers or their cross-linked forms.

Furthermore, arbitrary ingredients used in pressure-sensitive adhesives known in the art can be mixed in addition to the adhesive base. Examples of such arbitrary ingredients include antioxidants (e.g., dibutylhydroxytoluene), softeners (e.g., liquid paraffin, castor oil, cottonseed oil, palm oil, coconut oil, and lanolin), tackifiers (e.g., rosin-based resins, terpene-based resins, petroleum-based resins, and phenol-based resins), and inorganic fillers (e.g., zinc oxide, aluminum oxide, titanium dioxide, silica gel, magnesium oxide, iron oxide, and zinc stearate).

As shown in Figure 9, the array 1 is placed on in the holding member 21 such that the pressure-sensitive adhesive layer 21b is contacted with the undersurface (surface on a side not provided with the microprotrusions 3) of the base 2. Because of placing the array 1, the area of the holding member 21 is larger than the area of the array 1. The holding member 21 may have twice or more the area of the array 1. The array 1 is fixed to a predetermined position (at the substantially center of the holding member 21 in the examples of Figures 8 and 9) of the holding member 21 by the adhesive force of the pressure-sensitive adhesive layer 21b.

The release liner 22 is a substantially round member for covering and protecting the pressure-sensitive adhesive layer 21b and the array 1 until the array 1 is placed against the skin. The periphery of the release liner 22 is attached to the pressure-sensitive adhesive layer 21b, and a site (substantially central site in the examples of Figures 8 and 9) covering the array 1 is elevated thereabove. Thus, it is said that the release liner 22 is a convex-shaped liner. The rim of the release liner 22 is extends more outwardly than the rim of the holding member 21 in order to facilitate peeling from the pressure-sensitive adhesive layer 21b. Thus, the area of the release liner 22 is larger than the area of the holding member 21. A portion of the rim of the release liner 22 is larger than the other portion and is a projecting part 22a that extends in a chevron pattern. A user can easily peel off the release liner 22 by grasping this projecting part 22a.

In this context, a thickness of 0.5 to 3 mm may be imparted to the base 2 for incorporating the array 1 to such a device 20.

Although the device 20 described using Figures 8 and 9 comprises the holding member 21 having the pressure-sensitive adhesive in order to continue to place the array 1 against the skin (in other words, in order to maintain the contact of the array 1 with the skin), the constitution of the device or the method for placing the array 1 against the skin is not limited to this. For example, the array provided with microprotrusions may be installed in a stretching instrument; the array provided with microprotrusions may be attached to the tip of a pressing instrument; or the array provided with microprotrusions may be attached to a fingerstall. Alternatively, the array provided with microprotrusions may be placed against the skin directly by a finger or the like or by a finger with a fingerstall without such installation.

**Examples**

Hereinafter, Examples of an array provided with microprotrusions or a device having the array will be described specifically, and however, the constitution of the array provided with microprotrusions and the constitution of the device having the array are not limited to Examples below.

(Example 1) Primary rabbit skin irritation test

<Operational procedure>

Three types of arrays provided with microprotrusions made of polylactic acid in which the lengths of the protrusions differed (h: 70 $\mu$m, 110 $\mu$m, and 150 $\mu$m), and an array consisting only of a base were used. With regard to three types of arrays provided with microprotrusions, all the shapes of the protrusions were quadrangular pyramids, and all the densities of the protrusions were 841 protrusions/cm$^2$. A test substance was pressed for 5 seconds with a force of 3 kg against the skin in the shaved dorsal portion of each 18-week-old female Japanese white rabbit (Kbl:JW) and then applied for 2 hours (applied using a tape in which an acrylic pressure-sensitive adhesive (Durotak 2194) was stacked on a polyethylene terephthalate film as a support) (the number of individuals n = 6). Then, the test substance was peeled off after 2 hours from administration, and erythema/eschar and edema formation were grossly observed on 0.5, 2, 24, 48, and 72 hours after peeling and graded on the basis of the scoring criteria of Draize et al. (Table 1).

The assessment of primary skin irritation was performed by calculating a primary irritation index (P.I.I.) and using the scoring criteria of Draize (Table 2) shown below. The primary irritation index was calculated by respectively determining the average scores of each individual as to erythema and edema formation after 0.5 hours and 24 hours after test substance (MN) peeling, further determining the total sum of the average scores of each group, and dividing by the number of individuals.

As shown in Table 3, the primary irritation index (P.I.I) of the skin was 0.0 for all of three types of arrays provided with the respective microprotrusions (70 $\mu$m, 110 $\mu$m, and 150 $\mu$m) and the array with only a base.

[Table 1]

Skin reaction scoring criteria (Draize)

| Erythema and eschar formation | Score |
| --- | --- |
| No erythema | 0 |
| Very slight erythema (barely perceptible) | 1 |
| Well-defined erythema | 2 |
| Moderate to severe erythema | 3 |
| Severe erythema (beet redness) to slight eschar formation (injuries in depth) | 4 |
| Edema formation | Score |
| No edema | 0 |
| Very slight edema (barely perceptible) | 1 |
| Slight edema (perceptible edges of area well defined by definite raising) | 2 |
| Moderate edema (raising approximately 1 mm) | 3 |
| Severe edema (raising approximately 1 mm and extending beyond the area of exposure) | 4 |

[Table 2]

Scoring criteria of primary skin irritation (Draize)

| P.I.I. $\leq$ 2 | Mild irritant |
| --- | --- |
| 2 < P.I.I. $\leq$ 5 | Moderate irritant |
| 5 < P.I.I. $\leq$ 8 | Severe irritant |

[Table 3]

| | N | 30 minutes after MN removal | | 24 hours after MN removal | |
| --- | --- | --- | --- | --- | --- |
| | | Erythema | Edema | Erythema | Edema |
| Base | 6 | 0.0 | 0.0 | 0.0 | 0.0 |
| 70 $\mu$m | 6 | 0.0 | 0.0 | 0.0 | 0.0 |
| 110 $\mu$m | 6 | 0.0 | 0.0 | 0.0 | 0.0 |
| 150 $\mu$m | 6 | 0.0 | 0.0 | 0.0 | 0.0 |

(Example 2) Rabbit skin occult blood test

<Operational procedure>

An array provided with microprotrusions made of polylactic acid in which the length of the protrusions was 150 $\mu$m was used. In this array, the shape of the protrusions was a quadrangular pyramid, and the density of the protrusions was 841 protrusions/cm$^2$. Similarly to Example 1 above, the array was pressed against the rabbit skin and applied for 2 hours (applied using a tape in which an acrylic pressure-sensitive adhesive (Durotak 2194) was stacked on a polyethylene terephthalate film as a support), and then, occult blood reaction was assayed (the number of individuals n = 6). Judgment criteria for occult blood were judged through color reaction when 20 $\mu$L of saline was added dropwise to the application site immediately after removing the array (MN) and test paper was placed thereagainst. Pretest II (urine occult blood test paper, manufactured by Wako Pure Chemical Industries, Ltd.) was used as a test paper for assay. The skin was abraded using an 18-G needle as a positive control and tested similarly.

The occult blood reaction of the protrusion-applied group was observed in none of the rabbits, and the occult blood reaction was negative. On the other hand, with regard to the abraded group, evident coloring (blue) was observed with the test paper, though bleeding was hardly observed grossly. Since the occult blood reaction was not observed as to the microprotrusions of 150 μm in height, it was expected that the occult blood reaction was not observed as to the microprotrusions of 150 μm or smaller in height used in the irritation test.

[Table 4]

|  | Animal No. | Immediately after MN removal |
|---|---|---|
|  |  | Occult blood reaction |
| 150 μm | 1 | None |
|  | 2 | None |
|  | 3 | None |
|  | 4 | None |
|  | 5 | None |
|  | 6 | None |

(Example 3) Rabbit transepidermal water loss (TEWL) assay

<Operational procedure>

Three types of arrays provided with microprotrusions made of polylactic acid in which the lengths of the protrusions differed (h: 70 μm, 110 μm, and 150 μm), and an array consisting only of a base were used. With regard to three types of arrays provided with microprotrusions, all the shapes of the protrusions were quadrangular pyramids, and all the densities of the protrusions were 841 protrusions/cm$^2$. Similarly to Example 1 above, each array was pressed against the rabbit skin, and water loss was assayed around 2 hours after pressing. Transepidermal water loss (TEWL) is an index for assaying the barrier function of the skin, and it has been shown that water loss from a damaged site increases when the barrier structure of the stratum corneum gets damaged.

The assay of transepidermal water loss was conducted before application of the array and after a few minutes from peeling and performed using VapoMeter (manufactured by Dlefin). An array provided with microprotrusions of 500 μm in height with a similar shape was used for a positive control group, and an array consisting only of a base was used for a negative control group.

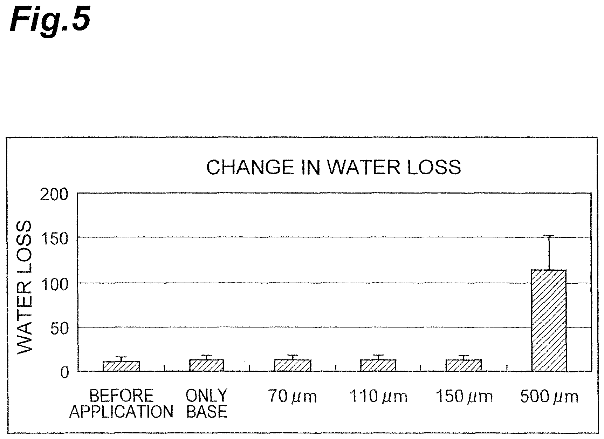

The experimental results are shown in Figure 5. In the case of using the array provided with microprotrusions of 500 μm in height, an evident rise in water loss was confirmed. On the other hand, although slight increase in water loss was observed for the arrays provided with microprotrusions of 70 to 150 μm in height, it was shown that the microprotrusions did not have influence on water loss because a similar rising tendency was also observed for the array not provided with microprotrusions.

From these results shown in Figure 5, it was assumed that the arrays provided with microprotrusions of 70 to 150 μm in height did not give physical damage to the skin under this condition.

(Example 4) Excised human skin impedance assay

<Operational procedure>

Three types of devices having arrays provided with microprotrusions made of polylactic acid in which the lengths of the protrusions differed (h: 70 μm, 110 μm, and 150 μm), and a device having an array consisting only of a base were used. All the shapes of the protrusions were quadrangular pyramids, and all the densities of the protrusions were 841 protrusions/cm$^2$. Each device was pressed for 5 seconds at a pressure of 3 kg against excised human skin, and the impedance value of the skin was measured before and after pressing. The impedance value of the skin is an index representing the barrier function of the skin as in TEWL, and it has been shown that the impedance value decreases when the skin gets damaged.

The measurement of the skin impedance value was performed by placing a skin fragment (skin thickness: approximately 500 μm, 5 cm × 5 cm) onto a stainless base, placing a round nonwoven fabric of φ 14 in diameter impregnated with saline and a silver electrode of φ 12 in diameter onto the base application site, and connecting the stainless base and the silver electrode with an LCR meter (3522-50, Hioki E.E. Corp.) (measurement conditions: 1 V, 10 Hz). A device having an array provided with microprotrusions of 500 μm in height with a similar shape was used for a positive control group, and a device having an array consisting only of a base was used for a negative control group.

In this context, the preparation of each device comprising an array provided with microprotrusions will be shown below.

(Example 5) Preparation of device comprising holding member and array provided with microprotrusions

An array provided with microprotrusions made of polylactic acid in which the shape of the protrusions was a quadrangular pyramid and the length thereof was 150 $\mu$m (density of the protrusions was 841 protrusions/cm$^2$) was used. Furthermore, ellagic acid as an active ingredient and propylene glycol or hydroxypropylcellulose as a coating agent were coated onto the array. Subsequently, the array was placed on a tape (holding member) prepared by applying a rubber-based pressure-sensitive adhesive onto a polyolefin-based foam support (diameter: 2.7 cm), to prepare a device having an array provided with microprotrusions.

(Example 6) Preparation of device comprising holding member and array provided with microprotrusions

A device having an array provided with microprotrusions was prepared by a similar approach as in Example 5 except that arbutin was used instead of ellagic acid as an active ingredient.

(Example 7) Preparation of device having microprotrusion array to be used in administration method of applying serum to skin in advance

A serum containing 4-(4-hydroxyphenyl)-2-butanol as an active ingredient solution was applied at 80 $\mu$L (20 $\mu$L/cm$^2$) to the shaved dorsal portion of each guinea pig, and then, a device having an array provided with microprotrusions was prepared for the purpose of stretching the skin by the microprotrusions of the array. The array was made of polylactic acid as a material and was of 150 $\mu$m as the length of the protrusions, 841 protrusions/cm$^2$ as the density of the protrusions, and 1 cm$^2$ as the area, and for a device, the array was fixed onto a covering member (holding member) prepared by stacking a rubber-based pressure-sensitive adhesive layer onto a support (3 cm $\times$ 5 cm) made of nonwoven fabric. As a result, the effect of suppressing pigmentation attributed to UVB irradiation was observed from the 4th day of device application onward, and the effectiveness of the present device was confirmed.

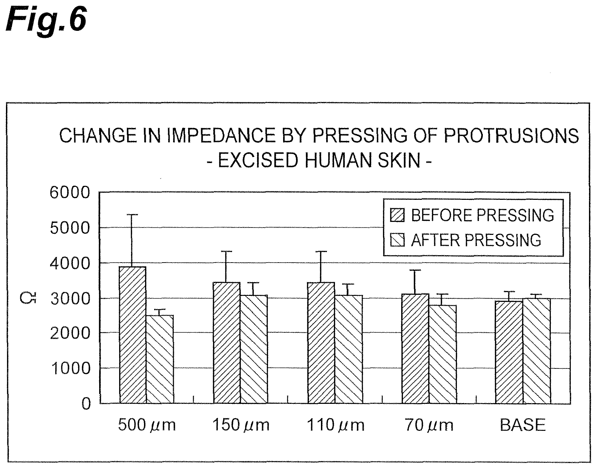

Returning to Example 4, the experimental results are shown in Figure 6. In the case of using the array provided with microprotrusions of 500 $\mu$m in height, the tendency that the impedance value decreased after pressing was observed. On the other hand, in the arrays provided with microprotrusions of 70 to 150 $\mu$m in height, correlation was not observed between the rate of decrease and the height, though a slight decreasing tendency was observed.

(Rate of stretching of skin in Examples 2 to 4)

<Case where skin was stretched completely along side of microprotrusion>

This case will be described using Figures 3(c) and 3(d). In the case where the skin with the length b along the line segment QM in a normal state has been stretched to the length a completely along the line segment PQ, the rate of stretching thereof depends on the apical angle $\alpha$ of the microprotrusion 3, regardless of the length thereof. As shown in Tables 5 to 7 below, the rates of stretching in the case where the apical angles $\alpha$ were 16, 18, and 20 degrees were 7.19, 6.37, and 5.74, respectively.

[Table 5]

| (Case where apical angle was 16 degrees) | | | |
|---|---|---|---|
| Length | b ($\mu$m) | a ($\mu$m) | Rate of stretching (a/b) |
| 150 $\mu$m | 21.11 | 151.87 | 7.19 |
| 100 $\mu$m | 14.07 | 101.22 | 7.19 |
| 50 $\mu$m | 7.04 | 50.65 | 7.19 |

[Table 6]

| (Case where apical angle was 18 degrees) | | | |
|---|---|---|---|
| Length | b (μm) | a (μm) | Rate of stretching (a/b) |
| 150 μm | 23.9 | 152.23 | 6.37 |
| 100 μm | 15.9 | 101.27 | 6.37 |
| 50 μm | 7.95 | 50.64 | 6.37 |

[Table 7]

| (Case where apical angle was 20 degrees) | | | |
|---|---|---|---|
| Length | b (μm) | a (μm) | Rate of stretching (a/b) |
| 150 μm | 26.56 | 152.33 | 5.74 |
| 100 μm | 17.71 | 101.56 | 5.74 |
| 50 μm | 8.85 | 50.78 | 5.74 |

<Case where skin was stretched incompletely along side of microprotrusion>

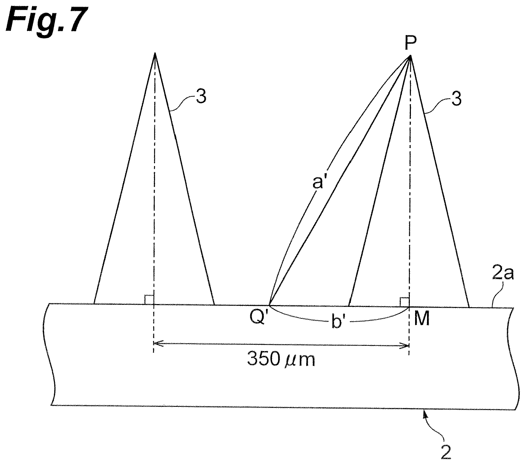

This case will be described using Figure 7. Figure 7 schematically shows the situation in which a portion of the skin was contacted with the base surface 2a between adjacent microprotrusions 3 by the pressing of the array provided with microprotrusions, so that the skin was not stretched along the side of the microprotrusion 3. If the skin is contacted with the base surface 2a at a midpoint Q' between the microprotrusions 3, the skin with a length b' along a line segment Q'M in a normal state shall be stretched to a length a' along a line segment PQ'. The rate a'/b' of stretching of the skin becomes a value shown in Table 8 below according to the height of the microprotrusions 3 provided that the microprotrusions 3 are arranged with equal spacing of 350 μm and the apical angle of the microprotrusions 3 is 20 degrees.

[Table 8]

| (Case where apical angle was 20 degrees) | | | |
|---|---|---|---|
| Length | b' (μm) | a' (μm) | Rate of stretching (a'/b') |
| 500 μm | 530.0 | 175 | 3.03 |
| 150 μm | 230.5 | 175 | 1.32 |
| 100 μm | 201.6 | 175 | 1.15 |
| 50 μm | 182.0 | 175 | 1.04 |

(Example 8) Evaluation of effect of potentiating skin-whitening effect by array provided with microprotrusions

When ultraviolet rays (UVB) are irradiated onto the shaved dorsal skin of each tortoiseshell guinea pig, pigmentation is induced. Change in brightness (ΔL*) in the case where an applied medicated whitening lotion was directly air-dried and change in brightness (ΔL*) in the case where an array provided with microprotrusions was applied to the skin after application of a medicated whitening lotion were compared with reduction in brightness (L*) caused by this pigmentation as an index. The change in brightness (ΔL*) was represented by the following formula:

$$[\text{Change in brightness } (\Delta L^*)] = [\text{Brightness before irradiation of ultraviolet rays}] - [\text{Brightness at the time of evaluation}]$$

The back of the tortoiseshell guinea pig was shaved, and UVB irradiation (260 mJ/cm$^2$) to the back was carried out three times on alternate days. Then, from a day following the day when the third UVB irradiation was performed, a medicated whitening lotion was applied to the back every day for 14 days. The tortoiseshell guinea pigs, which were test subjects, were divided into a group in which the applied medicated whitening lotion was directly air-dried and a group in which an array provided with microprotrusions (length of each microprotrusion: 150 μm, density of the microprotrusions: 841 protrusions/cm$^2$; made of polylactic acid) was applied for 4 hours after application of the medicated whitening lotion. Then, brightness was assayed at each time of before irradiation of ultraviolet rays, before the start of lotion application, during the lotion application period, and a day following the final day of lotion application, and the amount of change in brightness (ΔL*) from before irradiation of ultraviolet rays was calculated.

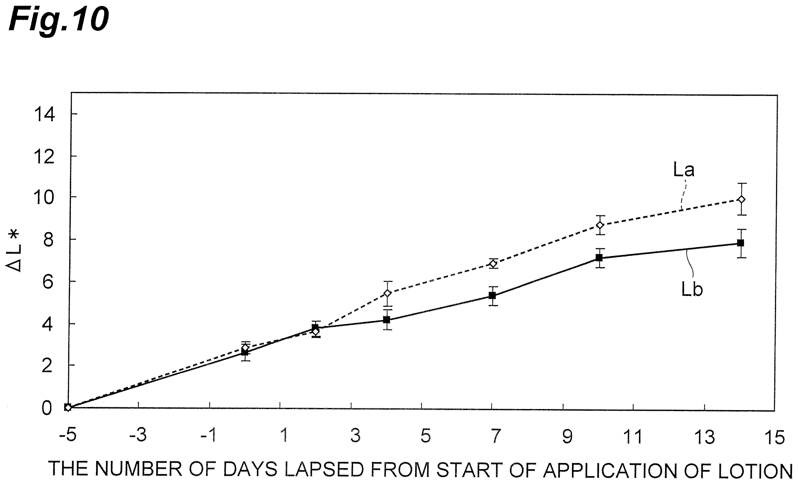

This result is shown in the graph of Figure 10. The abscissa of the graph is the number of days lapsed from the start of application of the medicated whitening lotion, and the ordinate is change in brightness (ΔL*). The broken line La represents change in brightness in the group in which the applied medicated whitening lotion was directly air-dried, and the solid line Lb represents change in brightness in the group in which the array provided with microprotrusions was applied to the skin after application of the medicated whitening lotion. As is evident from this graph, it was demonstrated that by applying the array provided with microprotrusions, change in brightness from before irradiation of ultraviolet rays was kept low, i.e., skin-whitening effect was enhanced.

(Example 9) Measurement of amount of sodium hyaluronate coating on microprotrusions

An array provided with microprotrusions made of polylactic acid in which the length of the protrusions was 150 μm was used. In this array, the shape of the protrusions was a quadrangular pyramid, and the density of the protrusions was 640 protrusions/cm$^2$.

Low-molecular-weight sodium hyaluronate (low-molecular HA) and various compounding agents were added to a 2-mL Eppendorf tube, and these were mixed to thereby prepare a coating solution. Each formulation relating to low-molecular HA is as shown in formulations 2 to 10 below. Moreover, a coating solution using high-molecular-weight sodium hyaluronate (high-molecular HA) was also prepared as a control formulation. The formulation relating to high-molecular HA is as shown in formulation 1 below.

(Formulation 1) high-molecular HA:water = 2:98
(Formulation 2) low-molecular HA:water = 15:85
(Formulation 3) low-molecular HA:pullulan:water = 15:5:80
(Formulation 4) low-molecular HA:trehalose:water = 15:5:80
(Formulation 5) low-molecular HA:marine collagen:water = 15:5:80
(Formulation 6) low-molecular HA:glycerin:water = 15:5:80
(Formulation 7) low-molecular HA:water = 10:90
(Formulation 8) low-molecular HA:trehalose:water = 10:10:80
(Formulation 9) low-molecular HA:marine collagen:water = 10:10:80
(Formulation 10) low-molecular HA:glycerin:water = 10:10:80

In addition, Red No. 40 was added to all of formulations 1 to 10 described above for coating content measurement. In all the formulations, the concentration of Red No. 40 was set to 1%.

Next, the array provided with microprotrusions was coated using each prepared coating solution. The range of coating was set to a range up to 100 μm from the tips of the microprotrusions. Then, the array provided with the coated microprotrusions was dipped in 1 mL of purified water and then extracted, and Red No. 40 in this water was quantified by HPLC (high-performance liquid chromatography) to thereby calculate the amount of sodium hyaluronate coating (n = 3). This calculation result is shown in the following table:

[Table 9]

| Formulation | HA molecular weight | HA concentration (%) | Additive (humectant, etc.) | Amount of HA coating (μg/patch) | Evaluation |
|---|---|---|---|---|---|
| 1 | High-molecular-weight HA Molecular weight: approximately 1 -2 × 10$^6$ | 2 | None | 1 μg or less | × |
| 2 | Low-molecular-weight HA Molecular weight: approximately 5 - 11×10$^4$ | 15 | None | 5.0 | ○ |

(continued)

| Formulation | HA molecular weight | HA concentration (%) | Additive (humectant, etc.) | Amount of HA coating (μg/patch) | Evaluation |
|---|---|---|---|---|---|
| 3 | Low-molecular-weight HA Molecular weight: approximately 5 - 11×10$^4$ | 15 | Pullulan (5%) | 2.6 | ○ |
| 4 | Low-molecular-weight HA Molecular weight: approximately 5 - 11×10$^4$ | 15 | Trehalose (5%) | 3.6 | ○ |
| 5 | Low-molecular-weight Molecular weight: approximately 5 - 11×10$^4$ | 15 | Marine collagen (6%) | 3.6 | ○ |
| 6 | Low-molecular-weight HA Molecular weight: approximately 5 - 11×10$^4$ | 15 | Glycerin (6%) | 5.1 | ○ |
| 7 | Low-molecular-weight Molecular weight: approximately 5 - 11×10$^4$ | 10 | None | 5.0 | ○ |
| 8 | Low-molecular-weight HA Molecular weight: approximately 5 - 11×10$^4$ | 10 | Trehalose (10%) | 5.3 | ○ |
| 9 | Low-molecular-weight Molecular weight: approximately 5 - 11×10$^4$ | 10 | Marine collagen (10%) | 7.1 | ○ |
| 10 | Low-molecular-weight HA Molecular weight: approximately 5 - 11×10$^4$ | 10 | Glycerin (10%) | 5.0 | ○ |

As a result, high-molecular HA could not sufficiently provide coating due to significantly low coating performance (adherence) to the microprotrusions, though viscosity occurred at a low concentration.

By contrast, low-molecular HA was prepared as approximately 10% aqueous solution to thereby cause sufficient viscosity, and from the result of coating content measurement, it was further shown that coating performance to the microprotrusions was also high. As a result of conducting verification by further increasing the concentration of low-molecular HA, it was demonstrated that this increase in concentration did not lead to increase in coating content, though thickening was observed.

**Industrial Applicability**

In an aspect of the present invention, since an active ingredient intended for beauty can be administered to the skin without pain and with reliability, with damage to the stratum corneum of the skin prevented, the convenience of the array provided with microprotrusions is drastically enhanced, and thus, there is industrial applicability.

**Reference Signs List**

1 ... Array provided with microprotrusions, 2 ... Base, 3 ... Microprotrusion, 4 ... Through-hole, 5 ... Coating, 20 ... Device having array provided with microprotrusions, 21 ... Holding member, 21a ... Support, 21b ... Pressure-sensitive adhesive layer, 22 ... Release liner.

**Claims**

1.    A device having an array provided with microprotrusions (20), comprising:

an array provided with microprotrusions (1), having a base (2) and tapered microprotrusions (3) each disposed on the base (2), each microprotrusion comprising a tip, a bottom and a surface, and tapering down toward the tip from the bottom connected to the base (2); and

a holding member (21) for placing the array provided with microprotrusions (1) against the skin, wherein denoting the distance from the tip to the bottom on an arbitrary side of each of the microprotrusions as a and denoting the length of a second line segment obtained by projecting a first line segment representing the distance onto the base as b, the ratio a/b satisfies $1.0 < (a/b) \leq 7.5$, the height of the microprotrusions is 50 to 300 $\mu$m, and the tip is flat and the area of the tip is 20 to 600 $\mu$m$^2$,

wherein the microprotrusions (3) are made of polylactic acid, and

wherein an active ingredient is coated at least on a portion on the surfaces of the microprotrusions and/or on the base, said active ingredient being a low-molecular weight sodium hyaluronate with a molecular weight of 50,000 to 110,000.

2. The device having an array provided with microprotrusions (20) according claim 1, wherein the density of the microprotrusions (3) is 100 to 10000 protrusions/cm$^2$.

3. The device having an array provided with microprotrusions (20) according to claim 1 or 2, wherein
the holding member (21) has a support (21a) and a pressure-sensitive adhesive layer (21b) stacked on the support (21a), and
the array provided with microprotrusions (1) is placed on the pressure-sensitive adhesive layer (21b).

4. The device having an array provided with microprotrusions (20) according to any one of claims 1 to 3, wherein an administration using the array with microprotrusions (1) is a direct administration by hand pressing.

**Patentansprüche**

1. Vorrichtung (20) mit einer mit Mikrovorsprüngen versehenen Anordnung, umfassend:

eine mit Mikrovorsprüngen versehene Anordnung (1), die eine Basis (2) und sich verjüngende Mikrovorsprünge (3) aufweist, die jeweils auf der Basis (2) angeordnet sind, wobei jeder Mikrovorsprung eine Spitze, einen Boden und eine Oberfläche umfasst und von dem mit der Basis (2) verbundenen Boden in Richtung der Spitze sich verjüngt; und

ein Halteelement (21) zum Platzieren der mit Mikrovorsprüngen versehenen Anordnung (1) an der Haut, wobei, wenn der Abstand von der Spitze zu dem Boden auf einer beliebigen Seite von jedem von den Mikrovorsprüngen als a bezeichnet wird und die Länge eines zweiten Liniensegments, das durch Projizieren eines ersten Liniensegments, das den Abstand wiedergibt, auf die Basis erhalten wird, als b bezeichnet wird, das Verhältnis a/b $1{,}0 < (a/b) \leq 7{,}5$ erfüllt, die Höhe der Mikrovorsprünge 50 bis 300 $\mu$m beträgt, und die Spitze flach ist und die Fläche der Spitze 20 bis 600 $\mu$m$^2$ beträgt,

wobei die Mikrovorsprünge (3) aus Polymilchsäure gefertigt sind, und

wobei ein Wirkstoff auf wenigstens einen Bereich auf den Oberflächen der Mikrovorsprünge und/oder auf die Basis aufgetragen ist, wobei es sich bei dem Wirkstoff um ein Natriumhyaluronat von geringem Molekulargewicht mit einem Molekulargewicht von 50000 bis 110000 handelt.

2. Vorrichtung (20) mit einer mit Mikrovorsprüngen versehenen Anordnung gemäß Anspruch 1, wobei die Dichte der Mikrovorsprünge (3) 100 bis 10000 Vorsprünge/cm$^2$ beträgt.

3. Vorrichtung (20) mit einer mit Mikrovorsprüngen versehenen Anordnung gemäß Anspruch 1 oder 2, wobei das Halteelement (21) einen Träger (21a) und eine auf den Träger (21a) geschichtete Haftklebstoffschicht (21b) aufweist, und
die mit Mikrovorsprüngen versehene Anordnung (1) auf der Haftklebstoffschicht (21b) platziert ist.

4. Vorrichtung (20) mit einer mit Mikrovorsprüngen versehenen Anordnung gemäß einem der Ansprüche 1 bis 3, wobei eine Verabreichung unter Verwendung der Anordnung (1) mit Mikrovorsprüngen eine direkte Verabreichung durch Andrücken mit der Hand ist.

**EP 2 578 264 B1**

**Revendications**

1. Dispositif présentant un réseau muni de microprotubérances (20), comprenant :

un réseau muni de microprotubérances (1), présentant une base (2) et des microprotubérances effilées (3) disposées chacune sur la base (2), chaque microprotubérance comprenant une pointe, un fond et une surface, et s'effilant vers le bas vers la pointe du fond reliée à la base (2) ; et
un élément de maintien (21) pour mettre en place le réseau muni de microprotubérances (1) contre la peau, où en désignant la distance depuis la pointe vers le fond sur un côté arbitraire de chacune des microprotubérances comme a et en désignant la longueur d'un second segment de ligne obtenu par la projection d'un premier segment de ligne représentant la distance sur la base comme b, le rapport a/b satisfait l'équation $1,0 < (a/b) \leq 7,5$, la hauteur des microprotubérances est de 50 à 300 $\mu$m, et la pointe est plate et la surface de la pointe est de 20 à 600 $\mu$m$^2$,
où les microprotubérances (3) sont constituées de poly(acide lactique), et
où un ingrédient actif est revêtu au moins sur une partie des surfaces des microprotubérances et/ou sur la base, ledit ingrédient actif est un hyaluronate de sodium de bas poids moléculaire présentant un poids moléculaire de 50 000 à 110 000.

2. Dispositif présentant un réseau muni de microprotubérances (20) selon la revendication 1, la densité des microprotubérances (3) étant de 100 à 10 000 protubérances/cm$^2$.

3. Dispositif présentant un réseau muni de microprotubérances (20) selon la revendication 1 ou 2, où
l'élément de maintien (21) présente un support (21a) et une couche adhésive sensible à la pression (21b) empilée sur le support (21a), et
le réseau muni des microprotubérances (1) est placé sur la couche adhésive sensible à la pression (21b).

4. Dispositif présentant un réseau muni de microprotubérances (20) selon l'une quelconque des revendications 1 à 3, où une administration utilisant le réseau présentant des microprotubérances (1) est une administration directe par pression avec la main.

Fig.1

# *Fig.2*

# Fig.3

(a)

3

B          B

(b)

P

3

α

a

Q   b   M   R

(c)

3

D          D

(d)

P

3

α

a

Q   b   M   R

# Fig.4

(a)

(b)

(c)

# *Fig.5*

# *Fig.6*

CHANGE IN IMPEDANCE BY PRESSING OF PROTRUSIONS
- EXCISED HUMAN SKIN -

Ω (vertical axis): 0, 1000, 2000, 3000, 4000, 5000, 6000

Legend:
- ☑ BEFORE PRESSING
- ◩ AFTER PRESSING

Horizontal axis: 500 μm, 150 μm, 110 μm, 70 μm, BASE

Fig.7

*Fig.8*

20

22a

1

IX

IX

22

21

**Fig.9**

**Fig.10**

THE NUMBER OF DAYS LAPSED FROM START OF APPLICATION OF LOTION

EP 2 578 264 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2001506904 A
- JP 2004504120 A
- JP 2007089792 A
- JP 2007130417 A
- US 2008208134 A1
- JP 2004510535 A